# EUROPEAN PATENT APPLICATION

(11) **EP 1 376 444 A1**
(43) Date of publication of application: **02.01.2004**
(21) Application number: 02405538.6
(22) Date of filing: 27.06.2002
(51) Int. Cl.: G06F 19/00

(54) **System and method for conducting a clinical trial**

(71) Applicant: ABB RESEARCH LTD., 8050 Zürich (CH)
(72) Inventor: Fricker, Samuel, 8006 Zürich (CH); Sager, Patrick, 6315 Oberägeri (CH)
(74) Representative: ABB Patent Attorneys

(57) **Abstract**

The present invention provides a system and method for conducting a clinical trial. The system includes a central clinical administration authority (CCTA) connected to a plurality of clinical trial centers (CTC) over a communicating means. Test persons are allocated clinical materials at the CTC. The CCTA administrates and coordinates the conduct of the clinical trial over these CTC. Clinical trial data (data relating to test persons and clinical materials) is stored in databases in each of the CTC. Each CTC has a software module that enables the exchange of trial administration data (data relating to administrating and coordinating the clinical trial, e.g. inventory levels of the clinical material in CTC, data regarding allocation of clinical materials to test persons) between the CTC and the CCTA on the basis of availability of the communicating means. The clinical trial data is stored in the CTC in encrypted format. The CCTA provides decryption keys to the CTC in order to decrypt the clinical trial data.

## Description

### Technical Area

The present invention relates to a system and method for conducting a clinical trial. More particularly, this invention relates to a system and method for conducting a clinical trial wherein clinical trial data is stored at clinical trial centers.

### State of Art Technology

When a pharmaceutical company launches a new drug into a market, it has to ensure that the drug is completely safe and does not harm target patients. Further, the drug should be at least as effective as other competing drugs in the market. There are regulatory bodies, such as the US Food and Drug Administration (FDA) that require the pharmaceutical companies to check the safety and efficacy of the drug before the drug is launched in the market. Pharmaceutical companies, thus, need to conduct a set of rigorous clinical trials before the launch of a new drug in the market.

As is evident from the above discussion, clinical trials are aimed at assessing the safety and efficacy of a new drug. These clinical trials, thus, involve monitoring various drug-assessing parameters. These drug-assessing parameters include effect of the drug on human metabolic and other activities, side effects of the drug on humans, and effectiveness of the drug vis-à-vis effectiveness of other therapies in the market.

Before the start of a clinical trial, preclinical research is carried out to assess the effect of the drug on animals and human cell cultures. If the drug passes this research phase, the phases of the clinical trial start. Typically, clinical trials for a new drug last for 8-10 years and involve three distinct trial phases as explained below.

Phase I Clinical Trials: Phase I trials are concerned with assessing drug tolerability, pharmacokinetics and pharmacodynamics and are typically carried out on a small set of healthy test persons. The phase I trials typically take several months to complete. If the drug passes this phase, the trials proceed to the next phase.

Phase II Clinical Trials: Phase II trials are concerned with assessing the efficacy and identifying the optimum dose of the drug with minimal side effects. Most phase II trials are randomised trials. In such randomised trials, one group of volunteers may be given the experimental drug while another "control" group will receive a placebo or a comparator. (A placebo is a tablet or capsule that contains no drug, but looks and tastes like a tablet or capsule that contains a drug). A test person enrolling for the trial will be randomly assigned to one of the two groups, namely the "drug" group or the "control" group. Neither the test person, nor the investigator does know if the drug or the placebo is being administered. In this manner, the psychological effects of taking a drug is accounted for, and the effect due to the action of drug can be measured effectively. Hence, the trials can assess and provide information about the effectiveness of the new drug.

Phase III Clinical Trials: Phase III trials involve testing the drug on several hundred to several thousand patients. This large-scale testing provides a more thorough understanding of the drug's effectiveness, benefits, and the range of possible adverse reactions or side effects, based on a 'real-life' population of patients. This helps in "fine tuning" dosage amounts and procedures to ensure safety and effectiveness of the drug. Most phase III trials are also randomised and blinded trials.

The scale, duration and assessment parameters of a clinical trial are governed by the phase, which the trial belongs to, and the trial protocol. For instance, a phase III trial will be larger in scale when compared to a phase I trial. However, the method of conducting a clinical trial (e.g. design, planning, clinical material production, logistics, investigation and analysis) does not vary much from one phase to another. The investigation stage of a clinical trial begins with the arrival of clinical material such as new drugs, placebos and comparators at various trial sites. The patients (test persons) for the clinical trial are selected on the basis of given eligibility criteria. These criteria may include various characteristics of test person such as disease history, age, sex and other demographic and medical characteristics. These test persons are given an identity card having an identification code. During the clinical trial, when a test person goes to a trial site, the identification code (on the identity card) is authenticated and a suitable clinical material is allotted to him/her. The clinical material is allotted to the test person on the basis of properties of the clinical material and the characteristics of the test person. During the whole process of clinical trial, data regarding the effect of the clinical material on test persons is collected and analysed at predefined intervals to determine the efficacy of the new drug.

During the clinical trial, it is imperative that the properties of the clinical material are matched with the characteristics of test person. The properties of the clinical material may include unique identification number (such as batch number), weight, clinical material type etc. The characteristics of the test person may include age, weight, unique identification number (such as social security number), clinical material type assigned to the test person etc. An inaccurate match of clinical material and test persons may jeopardize the results of the clinical trial as well as potentially prove harmful for the test person. The exact match of the properties of drug and characteristics of test person is required for precise evaluation of these drugs for different sets of conditions. For instance, a drug with high potency may prove to be more effective, if given to a person having a disorder of higher severity.

Besides the placebo effect for a patient, there is also a distinct possibility of bias in the judgement of effects of drugs on the part of participants of the trial. For instance, a clinical investigator may have a bias in judging the effect of a drug if he/she is aware of the clinical material administered to a test person. Thus, secrecy of the clinical data (including certain patient and certain clinical material characteristics) has to be maintained to prevent any bias from the participants of the trial i.e. patients, investigators and sponsors. Various techniques, such as randomisation (described above) and blinding, are used to maintain this confidentiality. Blinding is a technique wherein the information about the kind of clinical treatment being given to a person, is kept confidential. Effective blinding of clinical material requires the blinding of the packaging, strength and composition of clinical material. Blinding also involves maintaining secrecy about information relating to patients involved in the clinical trial. Effective blinding of test persons requires the blinding of at least the type of clinical material that should be consumed.

It is imperative that the properties of clinical material are matched with the characteristics of the test person. At the same time, it is also imperative to maintain a high degree of confidentiality regarding the properties of clinical material and the characteristics of test persons. Achieving both the objectives at the same time in an efficient manner is a difficult task.

One known way of accounting for matching and respecting confidentiality is performing an "in-advance" matching. "In-advance" matching technique assigns a test person for the clinical material in advance. The test person as well as the investigating personnel are, however, unaware of the kind of clinical material he/she is being administered. Thus, an appropriate clinical material is assigned to the test person and confidentiality is maintained at the same time. However, "in-advance" matching suffers from a drawback. In case a test person drops out from the clinical study, the unused clinical material assigned to him/her at the beginning of the trial cannot be re-allocated to any other test person because of the secrecy requirement on clinical material and patients. Therefore, this unused clinical material is discarded thereby leading to a high degree of clinical material wastage.

Certain existing patents seek to provide a solution to the abovementioned problem but suffer from a few drawbacks. EP 0 710 917 A1 titled 'Method and Apparatus for administering Clinical trial material' describes a method and an apparatus for administering clinical material to test persons. The clinical investigator first identifies a test person and contacts a computer over a telephone to identify the appropriate clinical material that is to be administered to an identified test person. The computer carries out a match to identify the clinical material to be administered to the test person. The computer has support capabilities for storing and disseminating information over the telephone. The clinical investigator then needs to communicate and confirm the information transmitted to him/her from the computer using the telephone. However, the disclosed system suffers from a drawback. In the disclosed system, the data is stored and disseminated from the computer using telephonic capabilities. If, however, the computer is not available for any reason, then the whole process of clinical trial gets affected. Thus, the computer and some communicating means have to be always available for clinical trials. Further, in case of a system crash, the whole data may be lost thereby making the whole process of clinical trials futile. There is also a possibility that the information being transferred from test site to central computer may be accessed by certain unscrupulous elements, such as hackers.

Another relevant system is disclosed in patent EP 0 999 506 A1 titled 'Method and system for dispensing, tracking and managing clinical trial products'. The system links prescribers and pharmacies using a central computing station that includes a database. A product trial media card, encoded with information that identifies a particular pharmaceutical product, is used instead of the actual pharmaceutical product. The prescriber matches and then prescribes a product by activating and dispensing the product trial media card to the patients. The participating pharmacies validate the media card by linking to the central computing station. These pharmacies then update their databases and dispense the pharmaceutical product. In this manner, all the pharmaceutical products can be accounted for. The drawback in the disclosed system is that the communication terminal at pharmacies is communicatively linked to the central computing station. Thus, the central computing station has to be always available for operation. A crash of central computing station would lead to breakdown of the whole system. There is also a possibility that the information being transferred from test site to central computer may be accessed by certain unscrupulous elements, such as hackers. Further, this invention makes the procedure complicated for the test person, as the test person has to go to the participating pharmacy with the product trial media card to get the prescribed product.

The existing patents, therefore, do not provide for a system for managing clinical trials effectively. Therefore what is required is a system that takes into account three major needs for conducting a clinical trial. Firstly, the system should ensure proper matching of test person characteristics with clinical material properties. Secondly, the data relating to the trial should be kept confidential to prevent any manipulation of data or results. Finally, the dependence of clinical trial sites on a central computer or a database should be minimized.

### Summary

It is an aim of the invention to provide a system for conducting a clinical trial wherein wastage of clinical material is minimized, a high degree of confidentiality is maintained and dependence of a clinical trial site on a central computer or a database is minimized.

To attain the above-mentioned aim, the system for conducting a clinical trial comprises of a plurality of investigation sites (hereafter referred to as clinical trial centers (CTCs)) to store and process clinical trial data, a central clinical trial administration (CCTA) to administrate and coordinate the plurality of CTCs, and a communicating means to enable data exchange between each of the plurality of CTCs and the CCTA.

In the preferred embodiment, the system for conducting clinical trial uses two layers of encryption - inner layer of encryption and outer layer of encryption, to secure the clinical trial data (data relating to clinical materials and test persons). The CCTA provides decryption keys (for both layers) to the CTC. When a test person arrives at the CTC, he/she is authenticated by the CTC. The CTC decrypts only the outer encryption layer of the encrypted test person code using the corresponding decryption key to obtain a set of encrypted test person characteristics. The CTC then looks through the list of codes of available clinical material. The outer encryption layer of each looked up code is decrypted using one of the decryption keys to obtain a set of encrypted clinical material properties. The set of encrypted clinical material characteristics is matched with the set of encrypted test person properties. As soon as matching clinical material has been found, the lookup process is stopped and matched clinical material is allocated to the test person. The CCTA is updated about the test persons and the matched clinical materials. The update of CCTA can take place on a periodic basis, for instance once a day.

### Short description of drawings

The preferred embodiments of the invention will hereinafter be described in conjunction with the appended drawings provided to illustrate and not to limit the invention, wherein like designations denote like elements, and in which:
FIG. 1 illustrates a system for conducting a clinical trial according to a preferred embodiment of the present invention;
FIG. 2 is a flowchart that shows a method for conducting a clinical trial according to the preferred embodiment of the present invention;
FIG. 3 illustrates an encryption process in which properties of a clinical material are encrypted into a clinical material code;
FIG. 4 illustrates an encryption process in which characteristics of a test person are encrypted into a test person code;
FIG. 5 illustrates a decryption process in which a clinical material code is decrypted to obtain properties of the clinical material;
FIG. 6 illustrates a decryption process in which a test person code is decrypted to obtain characteristics of the test person;
FIG. 7 is a flowchart that illustrates a method for allocating clinical material to test person in accordance with the preferred embodiment of the present invention;
FIG. 8 is a flowchart that illustrates a method for disclosing clinical trial data related to a test person, in case of a severe adverse event;
FIG. 9 is a flowchart that illustrates a method for disclosing clinical trial data related to a clinical material, in case of a severe adverse event.

### Steps to execute the invention

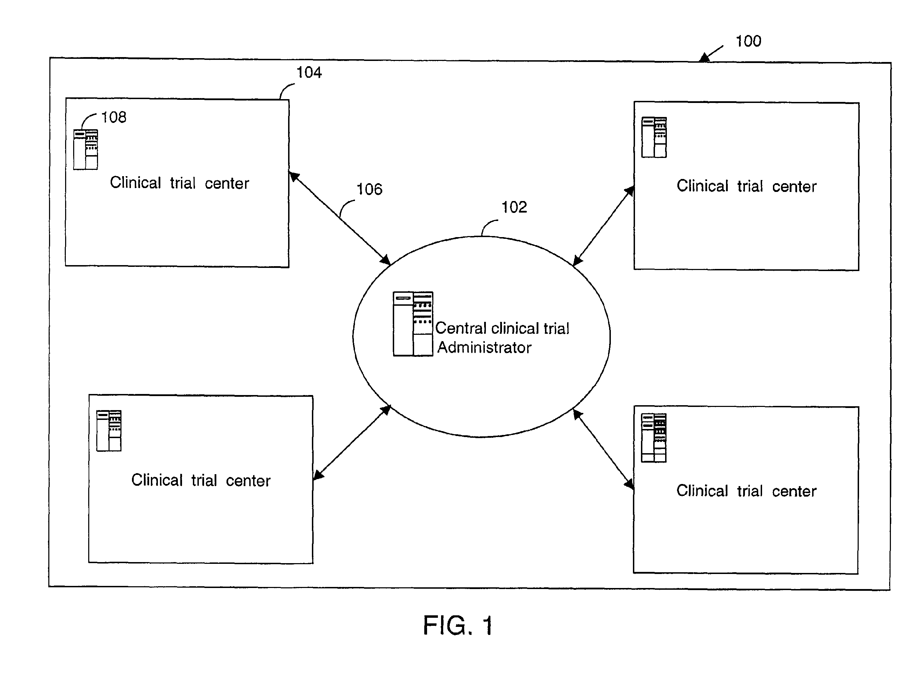

FIG. 1 illustrates a system 100 for conducting a clinical trial. System 100 comprises a Central clinical trial administration (CCTA) 102 connected to plurality of clinical trial centers (CTCs) 104 over a communicating means 106. CTC 104 allocates an appropriate clinical material to an enrolled test person, when the test person visits CTC 104. CCTA 102 administrates and coordinates the functioning of each such CTC 104. CCTA 102 may also manage inventories of each CTC 104 by using a Vendor Managed Inventory (VMI) solution or any standard clinical trial management software solution. In order to facilitate this administration of CTC 104, trial administration data is exchanged between CCTA 102 and CTC 104 over communicating means 106. The trial administration data is stored in CCTA 102 and is used for administrating the conduct of the trial at CTCs 104. This data includes a unique identifier (such as a serial number for CTC 104) that is used for identifying a given CTC 104 at CCTA 102. This data also includes data relating to inventory levels of a clinical material at CTC 104 and data relating to the allocation of a clinical material to a test person.

Each CTC 104 acts as an independent investigation site for carrying out the clinical trial. Each CTC 104 receives clinical material from one or more clinical material production sites before and during the clinical trial. CTC 104 also enrols test persons for participating in the trial before and during the clinical trial. The data relating to test persons and clinical materials are stored in CTC 104. This data is termed as clinical trial data. Note that clinical trial data stored in CTC 104 relates only to clinical materials and test persons specific to CTC 104. In a preferred embodiment, the clinical trial data is stored in the CTC 104 in an encrypted format. The encrypted test person code may be given to the test person in form of a test-person code on an identification card. During the clinical trial, CTC 104 allocates the clinical material to the enrolled test persons on the basis of properties of the clinical material and characteristics of the test person. Note that CTC 104 allocates a particular clinical material to the test person. This is unlike the process of randomisation, which involves allocating only the type of clinical material (for instance, drug, placebo or a comparator drug) to the test person.

Each CTC 104 includes a software module 108 for processing the clinical trial data. Software module 108, in turn, consists of an allocating module, a disclosing module and an updating module among other modules. The allocating module of CTC 104 allocates the clinical material to the test person. The updating module of CTC 104 updates CCTA 102 about various events at CTC 104 by exchanging the trial administration data. These events include arrival of clinical material at CTC 104, matching of a particular clinical material to a test person and other such events. The disclosing module of CTC 104 discloses the clinical trial data relating to the test person in case of a severe adverse event. The disclosed clinical trial data includes properties of the clinical material that have been allocated to and consumed by the test person. In such cases, the test person has to be blocked from undergoing further clinical trial as his/her characteristics and properties of the clinical material consumed by him/her have been disclosed. CTC 104 maintains a list of such blocked test persons. CTC 104 may also accommodate a decryption algorithm. The allocating module and the disclosing module may use this decryption algorithm for carrying out their tasks.

CCTA 102 manages the conduct of clinical trial across all CTCs 104. CCTA 102 stores decryption keys that are sent to CTC 104. In the preferred embodiment, CCTA 102 sends these decryption keys to CTC 104 at the start of a clinical trial. Alternatively, the decryption keys may also be sent to CTC 104 at predefined instants of time. CCTA 102 stores the clinical trial data that relates to all clinical materials and test persons involved in the clinical trial. CCTA 102 also stores trial administration data. The trial administration data includes data regarding allocation of clinical materials to each test person. This also includes data relating to the inventory levels of the clinical materials at each CTC 104. Such trial administration data is required to track the overall progress of the clinical trial across all CTCs 104. Preferably, CCTA 102 also stores a list of test persons who have been blocked from the clinical trial due to various reasons. One such reason may be that all the clinical trial data relating to them has been disclosed in a case of an adverse event.

CTC 104 is connected to CCTA 102 through a communicating means 106. In the preferred embodiment, communicating means 106 is implemented over a Wide Area Network (WAN). Alternatively, communicating means 106 may be implemented over a Local Area Network (LAN), Metropolitan Area Network (MAN) or the Internet. The physical medium for networks WAN, LAN, MAN or the Internet may be either wired or wireless. Preferably, an encrypted connection is used for improved security of data transmission between CCTA 102 and CTC 104. In order to achieve this, Transport Layer Security (TLS)/ Secure Sockets Layer (SSL) technology may be used. Alternatively, communicating means 106 may also be a physical data storage medium, such as magnetic diskettes, CDs, papers, etc.

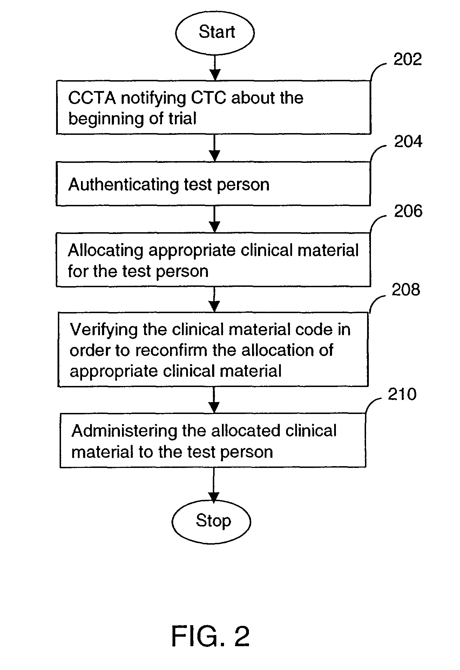

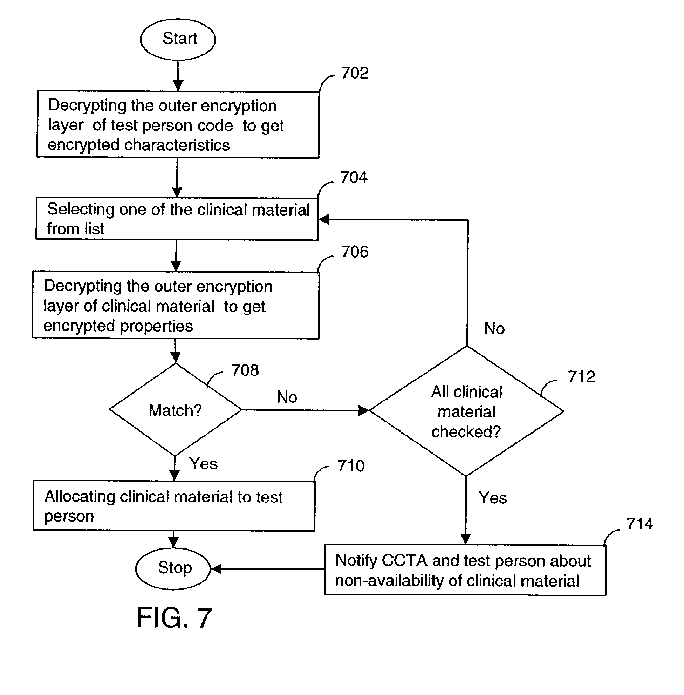

FIG. 2 is a flowchart that shows the sequence of steps to be followed in a method of conducting a clinical trial at CTC 104. Before and during the clinical trial, CTC 104 receives and registers new clinical materials for the clinical trial from one or more production sites. CTC 104 also enrols test persons for the clinical trial before and during the clinical trial. The characteristics of enrolled test persons and properties of received clinical materials are encrypted at CCTA 102 using any standard (synchronous or asynchronous) encryption scheme, such as RSA, Triple DES, IDEA etc. The encryption process generates clinical material codes and test person codes. These encrypted codes are then stored in CTC 104. In the preferred embodiment, the clinical material codes are used to label the outer packages of the clinical material and the test person code is put on an identification card that is assigned to each test person. At step 202, as soon as the clinical trial begins, CCTA 102 notifies CTC 104 about the beginning of the trial. In response to this notification, CTC 104 sends a unique identifier that enables CCTA 102 to identify CTC 104. Preferably, on receipt of this notification, CCTA 102 sends decryption keys to CTC 104 for decrypting the clinical trial data (data relating to test persons and clinical materials). Alternatively, this decryption key may also be sent to CTC 104 at pre-defined intervals, such as once a day. As soon as a test person arrives at CTC 104 for receiving the clinical material, CTC 104 authenticates the test person, at step 204, using the clinical trial data stored in CTC 104. The test person code is authenticated only if the test person is enrolled for the clinical trial and is not blocked by the system to undergo the clinical trial. This authentication may be done by various methods. In the preferred embodiment, the test person code can be authenticated using the clinical trial data stored in CTC 104. Alternatively, other authentication methods such as authentication of a biometric property (such as fingerprints) of the test person, password authentication of a user-specific username/password, etc. may also be used. Once the test person has been authenticated, at step 206, the allocating module allocates an appropriate clinical material to the test person. The search for an appropriate clinical material by the allocating module involves decrypting codes and checking for an appropriate match. The matching is done on the basis of characteristics of the test person and properties of the clinical material. The allocating module keeps on searching the clinical material codes and checking its match with the test person. The search for the clinical material ends when an appropriate match is found. The allocating module then assigns the appropriate clinical material to the test person. The search may also terminate if no matching clinical material is found. In that case, CTC 104 may notify CCTA 102 and the test person about the non-availability of the clinical material for the test person. CCTA 102 may then take appropriate administrative action, such as ordering clinical materials for CTC 104. The step of allocating the appropriate clinical material is described in greater detail in conjunction with FIG. 7. Before the test person can get the appropriate clinical material, at step 208, the clinical material code of the allocated clinical material is verified in order to reconfirm that appropriate clinical material is being assigned to the test person. This verification may be carried out in number of ways. One such way of verification is scanning the code of clinical material that is being allocated to the test person. If the scanned code is same as the code of allocated clinical material, the clinical material is verified. Alternatively, this verification may be carried out by manually entering the clinical material code in CTC 104. Once an appropriate clinical material has been assigned to the test person, at step 210, the appropriate clinical material is administered to the test person. The information about the match is forwarded to CCTA 102 by the updating means when communicating means 106 is available.

The abovementioned system and method for conducting a clinical trial use the clinical trial data in encrypted form. Note that elements of the described system and steps of the described method can be conditioned to different techniques of encryption. Furthermore, the elements of the described system and steps of the described method may also be conditioned for different levels of encryption, namely single encryption, double encryption, etc. We now describe an encryption process that will be used for encryption in the preferred embodiment of the invention. The elements of the system and the steps of the method for clinical trial will also be described in accordance with the disclosed encryption technique.

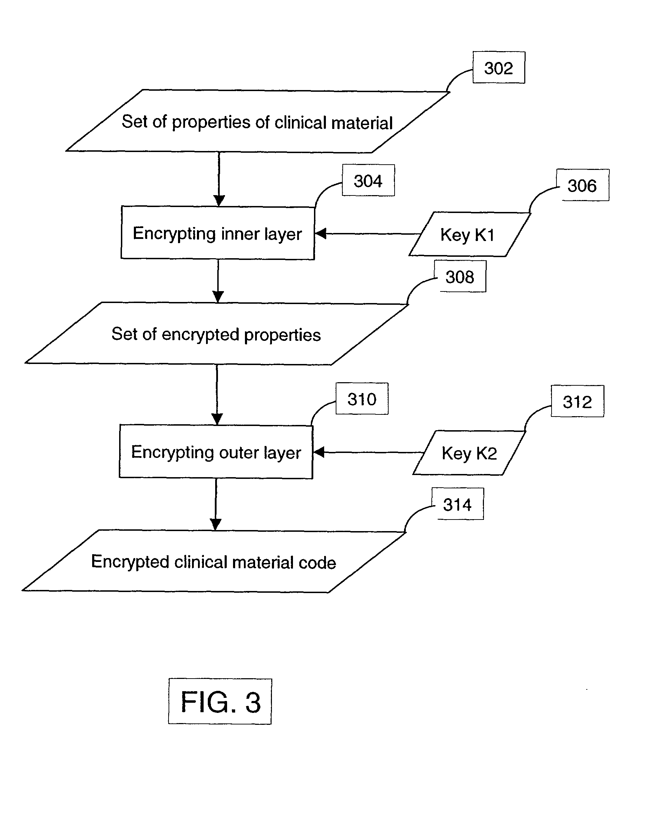

FIG. 3 illustrates an encryption process in which properties of a clinical material are encrypted into a clinical material code. The encryption process uses two layers of encryption - the inner layer of encryption and the outer layer of encryption. Each clinical material has a set of properties 302 associated with it. These properties may be production data like the series number or the date of production. These properties may also be medical, physical, material and/or clinical trial related properties like the type of clinical material. These properties could also be any other properties that help to identify the clinical material in some manner. Of all the properties that are encoded, at least one property should preferably be a unique identifier property (to guarantee the uniqueness of the code). This is to ensure that the clinical material can be identified uniquely. Another property of the clinical material, namely the clinical material type, is an essential property. This property is required for classification of the clinical material as a test drug with a specific concentration, a placebo or a comparator. Alternatively, a combination of properties that can uniquely identify a clinical material may also be used to generate a unique code. This code may then be used instead of the unique identifier property to uniquely identify the clinical material. The inner layer of encryption involves encrypting each property in set of properties 302 of clinical material. Thus, at step 304, inner layer encryption is done using a key K1 306. As a result of this inner layer encryption, a set of encrypted properties 308 of the clinical material is obtained. In the preferred embodiment, set of encrypted properties 308 is used in matching a clinical material with a test person. The outer layer of encryption involves encrypting set of encrypted properties 308. Thus, at step 310, outer layer encryption is done using a key K2 312. As a result, a clinical material code 314 is obtained. Once the code is generated, this code may be printed on the outer package of the clinical material as an alphanumeric code or a bar code 314. Alternatively, magnetic stripes, magnetic chips, etc. may also be used to identify the clinical material.

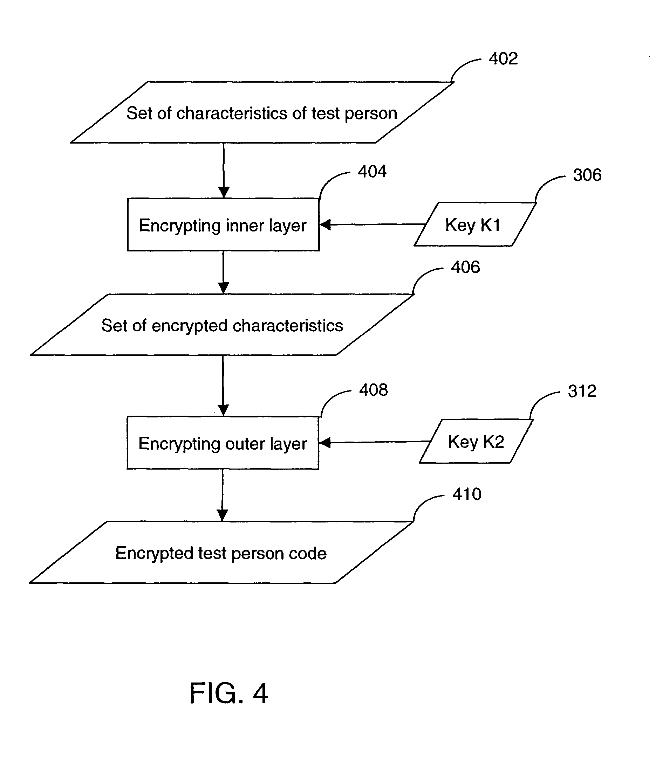

FIG. 4 illustrates an encryption process in which characteristics of a test person are encrypted into a test person code. Each test person has a set of characteristics 402 associated with him/her. This set may include physical characteristics such as birth date, legal characteristics such as social security number, medical characteristics such as relevant disease history and clinical trial characteristics such as planned consumption type. Again, at least one of these characteristics must preferably be a unique identifier characteristic that guarantees the uniqueness of the generated code. Alternatively, a combination of characteristics that can uniquely identify a test person may also be used to generate a unique code. This unique code may then be used instead of the unique identifier characteristic. The consumption type for a given test person must also be mentioned. This information is necessary to determine whether the person would consume a test drug at a specific concentration, a placebo or a comparator. The encryption process used in this case is same as the abovementioned encryption process for clinical material data. It uses two layers of encryption - the inner layer of encryption and the outer layer of encryption. At step 404, inner layer encryption of set of characteristics 402 is done using key K1 306 to obtain set of encrypted characteristics 406. In the preferred embodiment, set of encrypted characteristics 406 is used in identifying the appropriate clinical material for the test person. At step 408, outer layer encryption is done on set of encrypted characteristics 406 using key K2 312 to obtain test person code 410. In the preferred embodiment, keys K1 and K2 are used for encryption of clinical material properties as well as for encryption of test person characteristics. In the preferred embodiment, each test person is given a card that holds test person code 410. This test person code is used for authentication of the test person as a participant of a clinical trial. The test person code 410 is also used for allocation of the appropriate clinical material to the test person during the clinical trial.

The processes disclosed in FIG. 3 and FIG. 4 can be carried out over a system for conducting a clinical trial as described in FIG. 1. Such a system will, thus, include a set of encrypted characteristics-generating module and a test person code-generating module that generate the set of encrypted characteristics and the encrypted test person code, respectively, as described above. The system will also include a set of encrypted properties-generating module and a clinical material code-generating module that generate the set of encrypted properties and the encrypted clinical material code, respectively, as described above.

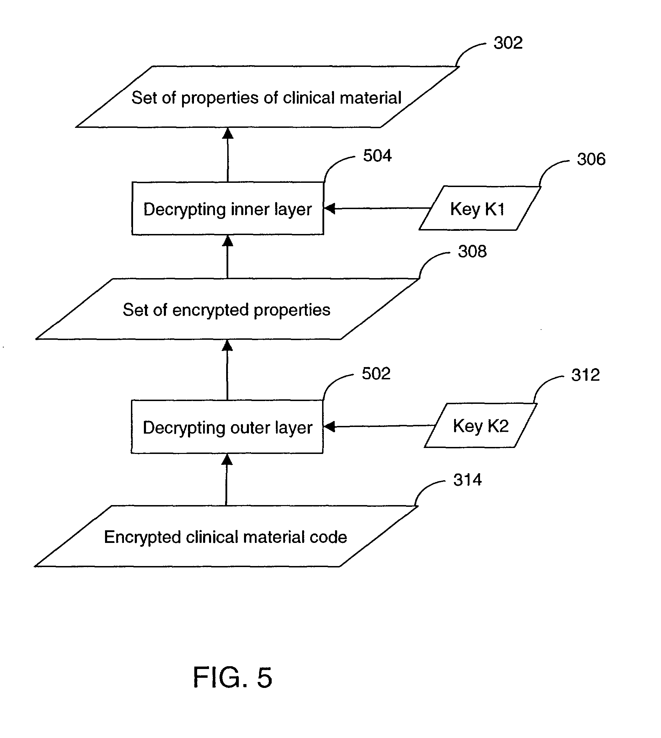

FIG. 5 shows a decryption process for decrypting encrypted clinical material code 314. At step 502, outer layer decryption of encrypted clinical material code 314 is done (using key K2 312) to obtain set of encrypted properties 308. At step 504, inner layer decryption of set of encrypted properties 308 is done (using key K1 306) to obtain set of properties of clinical material 302.

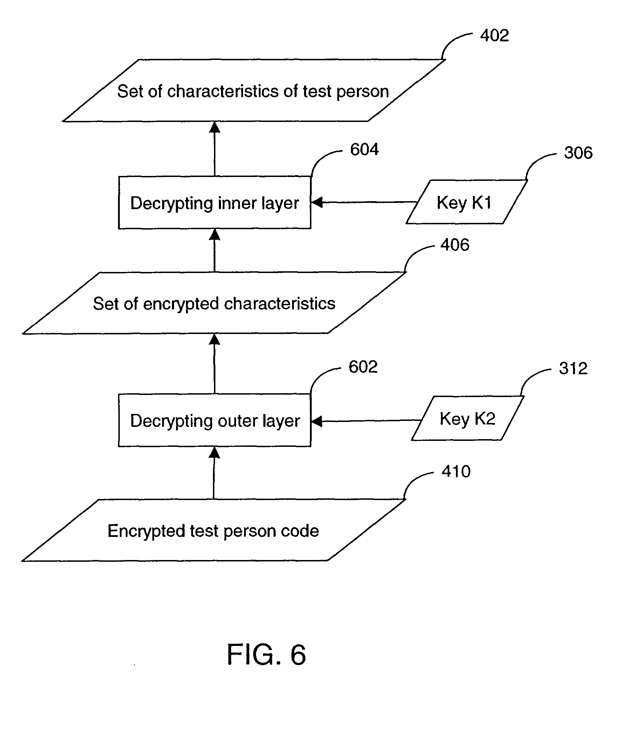

FIG. 6 shows a decryption process for decrypting encrypted test person code 410. At step 602, outer layer decryption of encrypted test person code 410 is done (using key K2 312) to obtain set of encrypted characteristics 406. At step 604, inner layer decryption of set of encrypted characteristics 406 is done (using key K1 306) to obtain set of characteristics of test person 402. In the preferred embodiment, keys K1 and K2 are used for decryption of clinical material codes as well as for decryption of test person codes.

Decryption processes described in FIG. 5 and FIG. 6 are used for decrypting the outer layer of test person codes and clinical material codes to obtain set of encrypted characteristics 406 of test person and set of encrypted properties 308 of clinical material. These are then used in allocation of clinical materials to a test person. Further, decrypting the inner layer to reveal the properties of clinical material and characteristics of test person will be used in revealing the clinical trial data relating to a test person, in case of a severe adverse event. The decrypting processes described in FIG. 5 and FIG. 6 is used in the decrypting algorithm that is accommodated in CTC 104 and is utilized by the allocating module and the disclosing module for carrying out their respective functions.

FIG. 7 illustrates the method of allocating clinical materials to a test person. When the test person arrives at CTC 104 to get the clinical material, at step 204, CTC 104 authenticates the test person. In the preferred embodiment, the test person code is entered into CTC 104 and is authenticated. At step 702, the outer encryption layer of the test person code is decrypted using decryption key K2 312 to get set of encrypted characteristics 406 of the test person. At step 704, the allocating module selects one clinical material code from the list of clinical material codes stored in CTC 104. This selection from the list can either be random or follow a definite pattern. This search is, of course, hidden and internal to the system. Thus, this information cannot be accessed and confidentiality is maintained. At step 706, the outer encryption layer of the selected clinical material code is decrypted using decryption key K2 312 to get set of encrypted properties 308 of the clinical material. At step 708, set of encrypted properties 308 is checked for matching with set of encrypted characteristics 406.This check for matching can be customised for different cases by defining the conditions for a match. As an example, one such condition for matching may be that the encrypted characteristic "consumption type" of the test person may be compared with the encrypted property "clinical material type" of the clinical material. A match can be defined to occur if the encrypted characteristic "consumption type" is exactly same as the encrypted property "clinical material type". If a match takes place, then at step 710, the matched clinical material is allocated to the test person. However, if the match does not take place, then at step 712, the allocating module checks if all the clinical materials have been checked for a match with the test person. If all clinical materials have not been checked for match, then the steps 704, 706 and 708 are repeated for other (unchecked) clinical material codes in the list. In case no clinical material matches the test person, at step 714, CTC 104 notifies CCTA 102 and the test person about the non-availability of clinical material.

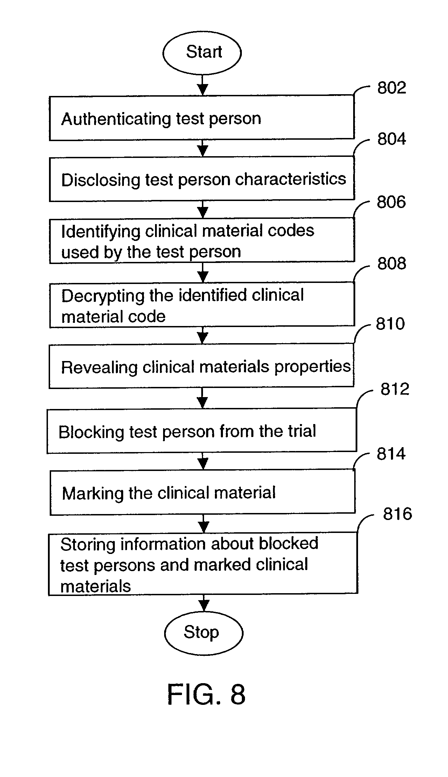

An adverse event may occur during the clinical trial if the test person reacts adversely after consuming the clinical material allotted to him/her by CTC 104. FIG. 8 shows a flowchart depicting the sequence of steps to be followed in case of such an adverse event. In such a situation, the disclosing module of CTC 104 gets activated. The disclosing module is used to disclose the properties of the clinical material consumed by the person. At step 802, the test person is authenticated. At step 804, the disclosing module discloses set of characteristics 402 of the authenticated test person by decrypting the test person code. This is done to ensure that the clinical trial data being revealed relates to the test person and not to any other test person. At step 806, the disclosing module identifies the codes of all clinical materials consumed by the test person. At step 808, the decryption module decrypts these clinical material codes and, at step 810, reveals set of properties 302 for these clinical materials. At step 812, the decryption module blocks the test person from participating in the trial and adds the test person to a list of blocked test persons. At step 814, it also marks all the revealed clinical materials. This marking may be done for a number of reasons. For instance, this marking may be used to obtain a list of clinical material whose properties have been disclosed in cases of adverse events. As soon as communicating means 106 is available, the updating module informs CCTA 102 about the blocked test persons as well as the marked clinical material. At step 816, the information about blocked test persons and marked clinical materials is stored.

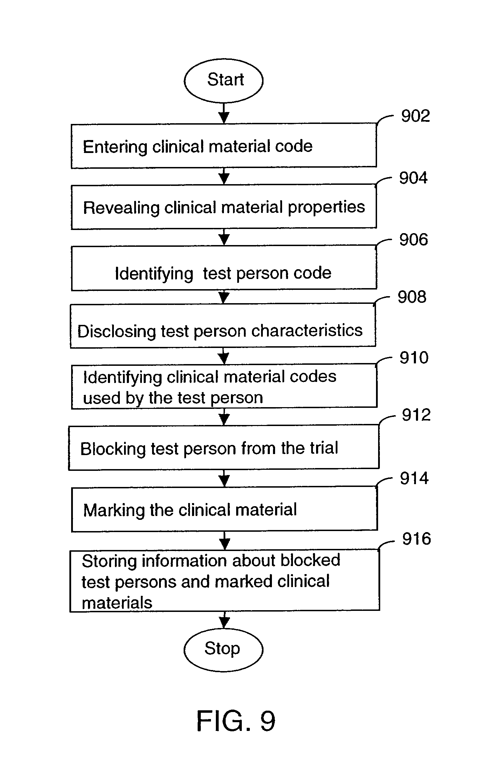

Alternatively, such adverse events may also occur if a clinical material undergoing the clinical trial is found to be unsuitable for test persons. A clinical material may be considered unsuitable for test persons for a number of reasons. One such reason may be that the consumption of the clinical material results in harmful side effects on test persons. In such a case, the test person who has been administered the clinical material has to be identified and an appropriate corrective action needs to be taken. FIG. 9 shows a flowchart depicting the sequence of steps to be followed in case of such a severe adverse event. In such a situation, the disclosing module of CTC 104 gets activated. At step 902, the clinical material code is entered into the system. At step 904, the disclosing module reveals set of properties 302 of the clinical material by decrypting the clinical material code. This is done to ensure that the clinical trial data being revealed relates to the clinical material and not to any other clinical material. At step 906, the disclosing module looks up test person codes to identify the test person, who consumed the clinical material. At step 908, the disclosing module decrypts this test person code and reveals set of characteristics 402 for this test person. At step 910, the disclosing module identifies codes of all clinical materials that have been consumed by the test person. The information about all the clinical material consumed by a test person may be necessary in determining the appropriate corrective action that is to be taken if a test person consumes an unsuitable clinical material. At step 912, the disclosing module blocks the test person from participating in the trial and adds the test person to a list of blocked test persons. At step 914, it also marks all the revealed clinical materials. This marking may be done for a number of reasons. For instance, this marking may be used to obtain a list of clinical material whose properties have been disclosed in cases of adverse events. As soon as communicating means 106 is available, the updating module informs CCTA 102 about the blocked test person as well as about the marked clinical material. At step 916, the information about the blocked test person and marked clinical materials is stored.

All the features disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features. For example, in an alternative embodiment the invention may use simple numbers/codes for identifying clinical materials and test persons instead of using encrypted codes for the same.

In an alternate embodiment, CCTA 102, apart from storing the trial administration data, also stores the clinical trial data in an encrypted form. When a test person arrives at the CTC 104 and enters the (encrypted) identification code, the authentication is done at CTC 104 itself. After authentication, CTC 104 sends the test person's code to CCTA 102. CCTA 102 then performs the decryption and allocation of the clinical material to the test person. After finding an appropriate match, CCTA 102 sends information about the matched clinical material to CTC 104. The CTC 104 then delivers the clinical material to the test person.

In another alternate embodiment, the system as described in FIG. 1 may use different outer layer decryption keys for outer layer decryption of clinical material codes and for outer layer decryption of test person codes. In this embodiment, the outer layer decryption key for test person code may be same for all CTCs 104 while that for clinical material codes may vary from one CTC 104 to another. Thus, CCTA 102 will contain only one outer layer decryption key for test person codes across all CTC 104. It will also have a plurality of outer layer decryption keys for clinical material codes, one outer layer decryption key for one CTC 104. This embodiment enables allocation of clinical material to test person at any CTC 104 involved in the clinical trial.

In yet another alternative embodiment, only sensitive properties of clinical material and sensitive characteristics of the test person are encrypted to generate the clinical material code and the test person code respectively. Non-sensitive properties and characteristics are not encrypted and remain in clear text. Thus, for a clinical material, properties such as type of the clinical material and strength of the clinical material are encrypted, while properties such as batch number, date of manufacture and date of expiry may be in unencrypted form. Similarly, for a test person, characteristics such as consumption type, age and disease history of the test person are in encrypted form, while characteristics such as social security number and geographical location are in unencrypted form. Thus, by selectively encrypting properties of clinical material and characteristics of test person, a desired degree of confidentiality regarding the clinical trial data can be obtained.

The previously described versions of the present invention have many advantages including: reducing clinical wastage, increasing security during the clinical trials and minimizing the dependence of clinical trial centers on the centralised authority. The invention does an in-advance assignment of clinical material type to the test person as opposed to the conventional in-advance assignment of the clinical material to the test person. According to the invention, the match of clinical material and test person is not constrained in advance; so that when a test person is excluded from a trial, other test persons (of the same kind) could consume the clinical material that the excluded test person was scheduled to consume. The invention uses encryption to code the clinical material and test person data to maintain a high degree of security. Moreover, the matching method provides flexibility and fairness to the conduct of trials.

The knowledge of this matching method lends flexibility to the trials since this method can be used to attain various degrees of blinding. For instance, if the matching method is not disclosed to the test person, then it is referred to as single blinding. If this matching method is not known to any of the participating parties, namely the test person, the clinical investigator or the sponsor, then it is referred to as triple blinding.

A high degree of fairness can be achieved in the process as well. This matching method can be defined by anybody (for instance FDA, or any other regulatory body) and the clinical trials can be carried out on anybody's terms or set of conditions.

The invention stores the clinical material and test person information in a distributed manner. Each of the clinical trial centers contains a database that stores the information. Also the matching of the clinical material to the test person is performed at the clinical trial center. This approach has significant advantage over the prior art solution that uses a central repository for storing and matching of data. In the present invention the CCTA does not need to be available at all times as opposed to the central repository solution. Furthermore, the system in the present invention would not lose all the information at a major system crash, unlike the central repository solution where a central system crash may result in loss of all information

While the preferred embodiments of the invention have been illustrated and described, it will be clear that the invention is not limited to these embodiments only. Numerous modifications, changes, variations, substitutions and equivalents will be apparent to those skilled in the art without departing from the spirit and scope of the invention as described in the claims.

## Claims

1. A system suitable for conducting a clinical trial, the system comprising:
a. a plurality of clinical trial centers, each clinical trial center comprising:
i. means for storing clinical trial data, the clinical trial data including data relating to test persons and clinical materials ; and
ii. means for processing the clinical trial data, the means for processing further comprising:
means for allocating a clinical material to a test person;
b. a central clinical trial administration for administrating the conduct of the clinical trial in the plurality of clinical trial centers, the central clinical trial administration storing trial administration data, the trial administration data including data relating to administration of the plurality of clinical trial centers; and
c. means for communicating between the central clinical trial administration and each of the plurality of clinical trial centers, the communicating means enabling the exchange of the trial administration data between the central clinical trial administration and each of the plurality of clinical trial centers,
whereby the central clinical trial administration administrates the plurality of clinical trial centers using the trial administration data obtained by the communicating means during the clinical trial.

2. The system as recited in claim 1, **characterized in that** the means for processing the clinical trial data further comprises:
a. means for registering the clinical trial data at the clinical trial center;
b. means for disclosing clinical trial data in case of a severe adverse event; and
c. means for updating the central clinical trial administration with the trial administration data on availability of the communicating means.

3. The system as recited in claim 1, **characterized in that** the means for allocating further comprises means for matching the clinical material and the test person.

4. The system as recited in claim 1, **characterized in that** the means for processing clinical trial data is embodied as a computer program.

5. The system as recited in claim 1, **characterized in that** the communicating means is either
at least one of Wide Area Network, Local Area Network, Metropolitan Area Network and Internet;
a wireless communicating means; or
a physical data storage.

6. A system suitable for conducting a clinical trial, the system comprising:
a. means for encrypting clinical trial data, the clinical trial data including data relating to test persons and clinical materials;
b. a plurality of clinical trial centers, each clinical trial center comprising:
i. means for storing the encrypted clinical trial data; and
ii. means for processing the encrypted clinical trial data, the means for processing comprising:
means for allocating the clinical material to the test person;
c. a central clinical trial administration for administrating the conduct of the clinical trial at the plurality of clinical trial centers, the central clinical trial administration storing trial administration data, the trial administration data including data relating to administration of the plurality of clinical trial centers; and
d. means for communicating between the central clinical trial administration and each of the plurality of clinical trial centers.

7. The system as recited in claim 6, **characterized in that** the means for encrypting clinical trial data further comprises:
a. means for generating a set of encrypted properties by encrypting a set of properties of the clinical material, the set of properties including a unique identifier property and a material type property;
b. means for generating an encrypted clinical material code by encrypting the set of encrypted properties;
c. means for generating a set of encrypted characteristics by encrypting a set of characteristics of the test person, the set of characteristics including a unique identifier characteristic and a consumption type characteristic, and
d. means for generating an encrypted test person code by encrypting the set of encrypted characteristics.

8. The system as recited in claim 6, **characterized in that** the means for processing the clinical trial data further comprises:
a. means for disclosing the clinical trial data relating to the test person in case of a severe adverse event; and
b. means for updating the central clinical trial administration with the trial administration data on availability of the communicating means.

9. The system as recited in claim 6, **characterized in that** the means for encrypting and/or the means for processing clinical trial data is embodied as a computer program.

10. The system as recited in claim 6, **characterized in that** the means for allocating further comprises means for matching the characteristics of test person and the properties of clinical material.

11. A system for securing clinical trial data in a clinical trial, the clinical trial data including data relating to clinical materials and test persons, the system comprising:
a. means for generating a set of encrypted properties by encrypting a set of properties of the clinical material, the set of properties including a unique identifier property and a material type property;
b. means for generating an encrypted clinical material code by encrypting the set of encrypted properties;
c. means for generating a set of encrypted characteristics by encrypting a set of characteristics of the test person, the set of characteristics including a unique identifier characteristic and a consumption type characteristic, and
d. means for generating an encrypted test person code by encrypting the set of encrypted characteristics
whereby the encrypted clinical material code and test person code is stored at a plurality of clinical trial centers, the clinical trial taking place at the plurality of clinical trial centers.

12. A method for securing clinical trial data in a clinical trial, the clinical trial data including data relating to clinical materials and test persons, the method comprising:
a. encrypting a set of properties of the clinical material to obtain a set of encrypted properties, the set of properties including a unique identifier property and a material type property;
b. generating an encrypted clinical material code by encrypting the obtained set of encrypted properties;
c. encrypting a set of characteristics of the test person to obtain a set of encrypted characteristics, the set of characteristics including a unique identifier characteristic and a consumption type characteristic, and
d. generating an encrypted test person code by encrypting the obtained set of encrypted characteristics.
whereby the clinical trial data is secured by encrypting the data relating to clinical materials and test persons to generate clinical material codes and test person codes.

13. A method for conducting a clinical trial, the clinical trial involving a central clinical trial administration, a plurality of clinical trial centers and a communicating means, each clinical trial center storing clinical trial data including data relating to clinical materials and test persons, the method comprising:
a. authenticating the test person at the clinical trial center;
b. identifying a clinical material code that matches test person code corresponding to the authenticated test person at the clinical trial center;
c. administering a clinical material corresponding to the identified clinical material code to the test person at the clinical trial center;
d. disclosing clinical trial data in case of severe adverse event at the clinical trial center; and
e. updating the central clinical trial administration with trial administration data on availability of the communicating means.

14. The method as recited in claim 13, **characterized in that** the step of authenticating the test person further comprises authenticating a biometric property of the test person.

15. The method as recited in claim 13, **characterized in that** the step of identifying a clinical material code comprises the steps of:
a. selecting one clinical material code from a list of available clinical material codes;
b. matching the clinical material and the test person, an allotment of clinical material taking place if the clinical material matches with the test person; and
c. repeating steps α-b for other available clinical material codes until the allotment of clinical material takes place.

16. A method for conducting a clinical trial, the clinical trial involving a central clinical trial administration, a plurality of clinical trial centers and a communicating means, each clinical trial center storing clinical trial data including data relating to clinical materials and test persons, the method comprising:
a. obtaining a decryption key at the clinical trial center from the central clinical trial administration;
b. authenticating the test person at the clinical trial center;
c. identifying a clinical material code that matches test person code corresponding to the test person authenticated at the clinical trial center;
d. administering a clinical material corresponding to the identified clinical material code to the test person at the clinical trial center;
e. disclosing clinical trial data in case of an adverse event; and
f. updating the central clinical trial administration with trial administration data.

17. The method as recited in claim 16, **characterized in that** the step of authenticating the test person code comprises checking whether the test person is blocked from undergoing the clinical trial.

18. The method as recited in claim 16, **characterized in that** the step of updating the central clinical trial administration comprises notifying the central clinical trial administration about the arrival of new clinical material at the clinical trial centers.

19. The method as recited in claim 16, **characterized in that** the step of updating the central clinical trial administration comprises notifying the central clinical trial administration about the allocation of the clinical material to test persons.

20. The method as recited in claim 16, **characterized in that** the step of updating the central clinical trial administration comprises notifying the central clinical trial administration about the disclosed clinical trial data.

21. The method as recited in claim 16, **characterized in that** the step of obtaining a decryption key comprises:
a. establishing communication between the central clinical trial administration and the clinical trial center over the communicating means;
b. identifying the clinical trial center at the central clinical trial administration; and
c. transferring a decryption key from the central clinical trial administration to the identified clinical trial center.

22. The method as recited in claim 16, **characterized in that** the step of identifying a clinical material code further comprises:
a. decrypting the test person code using a decryption key to obtain set of encrypted characteristics of the test person;
b. selecting a clinical material code from a list of available clinical material codes;
c. decrypting the clinical material code using the decryption key to obtain set of encrypted properties of the clinical material;
d. matching the set of encrypted characteristics of the test person and the set of encrypted properties of the clinical material;
e. allotting the clinical material to the test person if matching takes place; and
f. repeating steps b-d for other available clinical material codes if matching does not take place.

23. The method as recited in claim 16, **characterized in that** the step of disclosing the clinical trial data comprises:
a. authenticating test person at the clinical trial center;
b. decrypting the test person code using decryption keys to obtain the set of characteristics of test person;
c. disclosing the set of characteristics of the test person;
d. decrypting the clinical material codes related to the test person using decryption keys to obtain the set of properties for each clinical material; and
e. disclosing the set of properties of each clinical material whose codes have been decrypted.

24. The method as recited in claim 16, **characterized in that** the step of disclosing the clinical trial data comprises:
a. decrypting the clinical material code using decryption keys to obtain the set of properties for the clinical material;
b. disclosing the set of properties of each clinical material whose code has been decrypted;
c. decrypting the test person code related to the clinical material using decryption keys to obtain the set of characteristics of the test person; and
d. disclosing the set of characteristics of the test person.
